# EUROPEAN PATENT SPECIFICATION

(11) **EP 3 691 665 B1**
(45) Date of publication and mention of the grant of the patent: **24.04.2024**
(21) Application number: 18864783.8
(22) Date of filing: 04.10.2018
(51) Int. Cl.: A61K 35/747, A61K 31/702, A61K 31/718, A61K 45/06, A23L 33/125, A23L 33/135, A61P 31/04

(54) **A PHARMACEUTICAL COMPOSITION COMPRISING A PROBIOTIC AND A PREBIOTIC TO PREVENT ACQUISITION OF OR TREAT DRUG RESISTANT INFECTIONS**
PHARMAZEUTISCHE ZUSAMMENSETZUNG MIT EINEM PROBIOTIKUM UND EINEM PRÄBIOTIKUM ZUR VERMEIDUNG DES ERWERBS VON ARZNEIMITTELRESISTENTEN INFEKTIONEN
COMPOSITION PHARMACEUTIQUE COMPRENANT UN PROBIOTIQUE ET UN PRÉBIOTIQUE POUR PRÉVENIR L'ACQUISITION D'INFECTIONS PHARMACORÉSISTANTES OU LES TRAITER

(30) Priority: 04.10.2017 IN 201731035103
(43) Date of publication of application: 12.08.2020
(73) Proprietor: Chr. Hansen A/S, 2970 Hoersholm (DK)
(72) Inventor: MISRA, Pravas Ranjan, Bhubaneswar Odisha 751002 (IN)
(86) International application number: PCT/IB2018/057720
(87) International publication number: WO 2019/069266

(56) References cited:
- WO-A1-2012/108830
- US-B2- 7 101 565
- SHIM YOON HEE ET AL: "Antimicrobial activity of lactobacillus strains against uropathogens : Antimicrobial activity of lactobacillus", PEDIATRICS INTERNATIONAL, vol. 58, no. 10, 1 October 2016 (2016-10-01), pages 1009-1013, XP055804240, XX ISSN: 1328-8067, DOI: 10.1111/ped.12949 Retrieved from the Internet: URL:https://onlinelibrary.wiley.com/doi/pd fdirect/10.1111/ped.12949>
- Reuman P D: "Lack of effect of Lactobacillus on gastrointestinal bacterial colonization in premature infants - PubMed", Pediatr Infect Dis, 1 December 1986 (1986-12-01), XP055804200, Retrieved from the Internet: URL:https://pubmed.ncbi.nlm.nih.gov/309926 9/ [retrieved on 2021-05-12]
- DELERUE THIBAUD ET AL: "The potential role of microbiota for controlling the spread of extended-spectrum beta-lactamase-producing Enterobacteriaceae (ESBL-PE) in neonatal population", F1000RESEARCH, vol. 6, 25 July 2017 (2017-07-25), page 1217, XP055804238, DOI: 10.12688/f1000research.10713.1 Retrieved from the Internet: URL:https://www.ncbi.nlm.nih.gov/pmc/artic les/PMC5531162/pdf/f1000research-6-11552.p df>
- CHANDEL DINESH S. ET AL: "Changes in the Gut Microbiota After Early Administration of Oral Synbiotics to Young Infants in India", JOURNAL OF PEDIATRIC GASTROENTEROLOGY AND NUTRITION, vol. 65, no. 2, 1 August 2017 (2017-08-01) , pages 218-224, XP055803850, US ISSN: 0277-2116, DOI: 10.1097/MPG.0000000000001522 Retrieved from the Internet: URL:https://www.ncbi.nlm.nih.gov/pmc/artic les/PMC5524612/pdf/nihms844006.pdf>
- PANIGRAHI P et al.: "A randomized synbiotic trial to prevent sepsis among infants in rural India", Nature, vol. 548, no. 7668, 16 August 2017 (2017-08-16), page 407, XP055589412,

## Description

### FIELD

The present disclosure relates to a pharmaceutical composition comprising a probiotic and a prebiotic for preventing acquisition of and/or alleviating symptoms associated with infections by Extended-Spectrum Beta-Lactamases (ESBL) containing bacteria and other drug resistant pathogens in mammals. More particularly, the present disclosure relates to a composition comprising at least one *Lactillobacillus* specie(s) as a probiotic and at least one oligosaccharide as a prebiotic for preventing acquisition and/or alleviating symptoms associated with infections by Extended-Spectrum Beta-Lactamases (ESBL) containing bacteria and other drug resistant pathogens in mammals.

### BACKGROUND

Background description includes information that may be useful in understanding the present invention. It is not an admission that any of the information provided herein is prior art or relevant to the presently claimed invention, or that any publication specifically or implicitly referenced is prior art.

An infection is the inimical colonization of a host organism by foreign species. Frequently, the infecting organism attempts to utilize the host's resources to promote its own multiplication. Thereby, the infecting organism, or pathogen, may interfere with the normal functioning of the host and can lead to more infection related disorders that may have a varying severity and that may lead in the worst case to death. Bacterial infection can be treated by proper antibiotics. However, the selection of a proper medication requires defining the type of infection to be treated. Taking the wrong antibiotics in error for treating a specific non-viral infection won't treat the infection and may even be detrimental. Additionally, an extensive usage of antibiotics might contribute to the generation of antibiotic resistant infectious species. One means of preventing the rapid spread of drug resistant pathogens in humans is to significantly reduce antibiotic use. However, before prophylactic antibiotic use can be completely discontinued a suitable antimicrobial alternative must be available.

Antibiotic resistance is a serious worldwide public health problem and is on the rise. While antibiotic stewardship is just beginning to be instituted at a handful of major government medical centers, indiscriminate use of antibiotics continues in urban as well as rural areas. Resistant Gram-negative organisms, particularly extended spectrum beta lactamase (ESBL)-producing organisms, are of escalating clinical concern in developing countries. ESBLs are predominantly found in *Enterobacteriaceae* (Bradford PA: Extended-spectrum β-lactamases in the 21st century: characterization, epidemiology, and detection of this important resistance threat. Clin Micro Rev. 2001, 14(4):933-951) with *Escherichia coli* and *Klebsiella pneumoniae* being the two most common organisms to harbor the ESBL-encoding genes: SHV, TEM and CTX-M. These plasmid-borne genes confer the ability to hydrolyze extended spectrum cephalosporins and monobactams (Jacoby GA, Munoz-Price LS: The new β-lactamases. N Eng J Med. 2005, 352:380-391). Certain ESBL organisms have also acquired co-resistance to other antibiotics, including fluoroquinolones, aminoglycosides (Isendahl et al, Fecal carriage of ESBL-producing E. coli and K. pneumonia in children in Guinea-Bissau: A hospital-based cross-sectional study. PLoS One. 2010, 7(12) e51981; Guimaraes et al, Genetic detection of extended-spectrum β-lactamase-containing Escherichia coli isolates and vancomycin-resistant Enterococci in fecal samples of healthy children. Microb Drug Res. 2009, 15(3):211-216; Lautenbach et al Epidemiological investigation of fluoroquinolone resistance in infections due to extended spectrum β-lactamase-producing Escherichia coli and Klebsiella pneumonia. Clin Infect Dis. 2001, 33:1288-1294; Ben-Ami et al, A multinational survey of risk factors for infection with extended-spectrum β-lactamase-producing Enterobacteriaceae in nonhospitalized patients. Clin Infect Dis. 2009, 49:682-90) and recently to carbapenems as well (Yong et al Characterization of a new metallo-beta-lactamase gene, bla(NDM-1), and a novel erythromycin esterase gene carried on a unique genetic structure in Klebsiella pneumoniae sequence type 14 from India. Antimicrob Agents Chemother. 2009, 53:5046-3054; Pitout et al The latest threat in the war on antimicrobial resistance. Lancet Infect Dis. 2010, 10:578-9; Kamarasamy et al Emergence of a new antibiotic resistance mechanism in India, Pakistan, and the UK: a molecular, biological, and epidemiological study. Lancet Infect Dis. 2010, 10:597-602; Price et al Clinical epidemiology of the global expansion of Klebsiella pneumoniae carbapenemases. Lancet Infect Dis. 2013, 13:185-96), which are the mainstay in treatment of infections caused by ESBL-producing organisms.

There have been reports from India of a rise in ESBL-producing Gram-negative strains in tertiary care hospitals (Lal et al, Occurrence of TEM & SHV gene in extended spectrum β-lactamases (ESBLs) producing Klebsiella sp. isolated from a tertiary care hospital. Indian J Med Res. 2007, 125:173-178; Jemima et al Multiplex PCR for blaCTX-M & blaSHV in the extended spectrum beta lactamase (ESBL) producing Gram-negative isolates. Indian J Med Res. 2008, 128:313-317) including outbreaks in neonatal intensive care units (NICUs) (Jain et al, Prevalence of extended-spectrum b-lactamase-producing Gram-negative bacteria in septicaemic neonates in a tertiary care hospital. J Med Microbiol. 2003, 52: 421-425; Cartelle et al, Risk factors for colonization and infection in a hospital outbreak caused by a strain of Klebsiella pneumoniae with reduced susceptibility to expanded-spectrum cephalosporins. J Clin Microbiol. 2004, 42:4242-4249; Lebessi et al, Extended-spectrum β-lactamase-producing Klebsiella pneumonia in a neonatal intensive care unit in the high-prevalence area of Athens, Greece. J Clin Microbiol. 2002, 40:99-804; Royle et al, Outbreak of extended spectrum beta lactamase producing Klebsiella pneumoniae in a neonatal unit. Arch Dis Child Fetal Neonatal Ed. 1999, 80:F64-8; Venezia et al, Molecular epidemiology of an SHV-5 extended-spectrum beta-lactamase in enterobacteriaceae isolated from infants in a neonatal intensive care unit. Clin Infect Dis. 1995, 21:915-23; Jiang et al, Detection of extended-spectrum β-lactamases in clinical isolates of Pseudomonas aeruginosa. Antimicrob Ag Chemother. 2006, 50:2990-2995, Chandel et al, Extended-spectrum β-lactamase-producing Gram-negative bacteria causing neonatal sepsis in India in rural and urban settings. J Medical Micro. 2011, 60:500-507). The situation is considered worse when we see reports of such resistance in commensal bacteria in healthy individuals (Pallecchi et al, Detection of CTX-M-type β-lactamase genes in fecal Escherichia coli isolates from healthy children in Bolivia and Peru. Antimicrob Agents Chemother. 2004, 48(12): 4556-4561, Kothari et al, Community acquisition of β-lactamase producing Enterobateriaceae in neonatal gut. BMC Microbiology. 2013, 13:136-141, Rahman et al, High rates of intestinal colonization with extended-spectrum lactamase-producing Enterobacteriacae among healthy individuals. J Investig Med. 2011, 59:1284-1286; Luvsansharav et al, Prevalence of risk factors associated with fecal carriage of CTX-M β-lactamase-producing Enterobacteriaceae in rural Thai communities. J Antimicrob Chemother. 2012, 67:1769-1774). Although hospital-based studies show a high degree of acquisition and transfer of ESBL to other patients, there is paucity of data on the burden of ESBL in the community setting in general, and specifically in India. Probiotics are health promoting bacteria that have been examined as an alternative or supplement to antibiotic therapies (Saavedra JM, Clinical applications of probiotic agents. Am J Clin Nutr. 2001, 73:1147S-1151S). A recent study in mice demonstrated the potential benefit of probiotics against infections due to drug resistant S. *typhimurium* DT104 (Asahara et al, Protective effect of Lactobacillus casei strain Shirota against lethal infection with multi-drug resistant Salmonella enterica serovar Typhimurium DT104 in mice. J Appl Microbiol. 2011, 110:163-73). In humans, consumption of probiotic-containing cheese during treatment with amoxicillin-clavulanate was shown to reduce the emergence of drug resistant enterococci in feces (Bertrand et al, Effect of cheese consumption on emergence of antimicrobial resistance in the intestinal microflora induced by a short course of amoxicillin-clavulanic acid. J Appl Microbiol. 2007, 102:1052-9). To the best of the knowledge of inventors of the present disclosure, such activity of probiotics has not been reported in neonates and young infants.

Human milk is generally a food of choice for neonates and full-term infants because of its nutritional composition and immunologic benefits. The nutritional value of raw or conventionally-processed donor milk, however, varies and in most instances, is not sufficient to meet the needs of neonates and infants. In the recent past, certain strains of bacteria have attracted considerable attention because they have been found to exhibit valuable properties for man if ingested. In particular, specific strains of genera *Lactobacilli* and *Bifidobacteria* have been found to be able to colonize the intestinal mucosa, to reduce the capability of pathogenic bacteria to adhere to the intestinal epithelium, to have immunomodulatory effects and to assist in the maintenance of well-being. Such bacteria are called probiotics. Prebiotics are sugars that help probiotic bacteria grow better in the intestine. However, prebiotics themselves have not been shown to have such effect alone.

There is an ongoing need in the art to develop newer and efficacious pharmaceutical compositions for neonates, infants, children and adults to treat infections by Extended-spectrum beta-lactamases (ESBL) containing bacteria and other drug resistant pathogens. Need is also felt of compositions that also provides other benefits such as promoting gut maturation, enhancing gut health, enhancing protection later in life, promoting maturation of the immune system, contributing to support of natural defenses, and enhancing gut comfort while reducing crying time, cramps and/or colics.

### OBJECTS

It is an object of the present disclosure to provide a pharmaceutical composition including a combination of a prebiotic and a probiotic.

It is an object of the present disclosure to provide a pharmaceutical composition that find utility in prevention of acquisition of and/or treatment of infections by Extended spectrum beta-lactamases (ESBL) containing bacteria and other drug resistant pathogens.

It is another object of the present disclosure to provide a pharmaceutical composition that promotes gut maturation and enhances gut health and protection later in life in neonates and infants.

It is yet another object of the present disclosure to provide a pharmaceutical composition that promotes maturation of the immune system and contributes to support of natural defences to enhance gut comfort and reduces crying time, cramps and/or colics in neonates and infants.

Other objects of the present disclosure will be apparent from the description of the invention herein below.

### SUMMARY

The present disclosure relates to a pharmaceutical composition comprising a probiotic and a prebiotic for preventing acquisition of and/or alleviating symptoms associated with infections caused by an Extended-Spectrum Beta-Lactamases (ESBL) producing organism and other drug resistant pathogens in mammals. More particularly, the present disclosure relates to a composition comprising at least one *Lactillobacillus* specie(s) as a probiotic and at least one oligosaccharide as a prebiotic for preventing acquisition of and/or alleviating symptoms associated with infections by Extended-Spectrum Beta-Lactamases (ESBL) producing bacteria and other drug resistant pathogens in mammals.

An aspect of the present disclosure provides a pharmaceutical composition for preventing acquisition of or for alleviating symptoms associated with an infection caused by an Extended-Spectrum Beta-Lactamases (ESBL) producing organism in a subject, the composition comprising: a therapeutically effective amount of a probiotic; and an effective amount of a prebiotic.

In an embodiment, the probiotic comprises at least one *Lactobacillus* species. In an embodiment, the prebiotic comprises at least one oligosaccharide. In an embodiment, the subject is a human. In an embodiment, the Extended-Spectrum Beta-Lactamases (ESBL) producing organism comprises Extended-Spectrum Beta-Lactamases (ESBL) producing gram negative bacteria. In an embodiment, the probiotic comprises *Lactobacillus plantarum.* In the invention the probiotic comprises *Lactobacillus plantarum* strain ATCC202195. In an embodiment, the at least one oligosaccharide is selected from a group comprising (a) a fructo-oligosaccharide (FOS), (b) a pectin or a pectic polysaccharide, (c) a mannan, (d) a pentosan, a beta-glucan, an arabinan or a galactan, and (e) mixtures thereof. In an embodiment, the at least one oligosaccharide comprises the fructo-oligosaccharide. In an embodiment, the composition is administered by any of an oral route of administration and a parenteral route of administration. In an embodiment, the composition comprises 1-10 billion counts of cells of *Lactobacillus plantarum* strain ATCC202195 and 100-500 mg of the fructo-oligosaccharide.. In an embodiment, the composition comprises 1-5 billion counts of cells of *Lactobacillus plantarum* strain ATCC202195 and 150-350 mg of the fructo-oligosaccharide. In an embodiment, the composition further comprises at least one pharmaceutically acceptable excipient.

Another aspect of the present disclosure relates to a method of preventing acquisition of or alleviating symptoms associated with an infection caused by an Extended- Spectrum Beta-Lactamases (ESBL) producing organism in a subject in need thereof, the method comprising administering to the subject a therapeutically effective amount of a probiotic and an effective amount of a prebiotic. In an embodiment, the administering comprises oral or parenteral administration of said probiotic and said prebiotic. In an embodiment, the method comprises administering to the subject a composition comprising the therapeutically effective amount of the probiotic and the effective amount of the prebiotic. In an embodiment, the composition further comprises at least one pharmaceutically acceptable excipient. In an embodiment, the administering comprises simultaneous, sequential or intermittent administration of the therapeutically effective amount of the probiotic and the effective amount of the prebiotic. In an embodiment, the probiotic comprises at least one *Lactobacillus* species. In an embodiment, the prebiotic comprises at least one oligosaccharide. In an embodiment, the at least one oligosaccharide is selected from a group comprising (a) a fructo-oligosaccharide (FOS), (b) a pectin or a pectic polysaccharide, (c) a mannan, (d) a pentosan, a beta-glucan, an arabinan or a galactan, and (e) mixtures thereof. In an embodiment, the therapeutically effective amount of the probiotic comprises 1-10 billion counts of cells of *Lactobacillus plantarum* strain ATCC202195. In an embodiment, the effective amount of the prebiotic comprises 100-500 mg of the fructo-oligosaccharide. In an embodiment, the therapeutically effective amount of the probiotic comprises 1-5 billion counts of cells of *Lactobacillus plantarum* strain ATCC202195. In an embodiment, the effective amount of the prebiotic comprises 150-350 mg of the fructo-oligosaccharide.

In an embodiment, said prebiotic is selected from a group consisting of (a) an oligosaccharide, (b) a fructo-oligosaccharide ("FOS"), such as a soy fructo-oligosaccharide, inulin or banana fiber, (c) a pectin or pectic polysaccharide, (d) a mannan, such as guar gum, locust bean gum, konjac, or xanthan gum, (e) a pentosan, beta-glucan, arabinan and galactan, such as larch arabinogalactan, and (f) mixtures thereof.

The compositions of the present disclosure provide a myriad of health benefits including, but not limited to, promoting gut maturation, promoting maturation of the gut nervous system, enhancing gut health, enhancing protection later in life, promoting the maturation of the immune system, contributing to support of natural defenses, contributing to support growth, and enhancing gut comfort while reducing crying time, cramps and/or colics, fulfilling at least partially the nutritional requirements of neonates and infants.

### DETAILED DESCRITPION

The embodiments herein and the various features and advantageous details thereof are explained more fully with reference to the non-limiting embodiments that are illustrated in the accompanying figures and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The present disclosure relates to a pharmaceutical composition comprising a probiotic and a prebiotic for preventing acquisition of and/or alleviating symptoms associated with infections caused by an Extended-Spectrum Beta-Lactamases (ESBL) producing organism and other drug resistant pathogens in mammals. More particularly, the present disclosure relates to a composition comprising at least one *Lactillobacillus* specie(s) as a probiotic and at least one oligosaccharide as a prebiotic for preventing acquisition of and/or alleviating symptoms associated with infections by Extended-Spectrum Beta-Lactamases (ESBL) producing bacteria and other drug resistant pathogens in mammals.

An aspect of the present disclosure provides a pharmaceutical composition for preventing acquisition of or for alleviating symptoms associated with an infection caused by an Extended-Spectrum Beta-Lactamases (ESBL) producing organism in a subject, the composition comprising: a therapeutically effective amount of a probiotic; and an effective amount of a prebiotic.

In an embodiment, the probiotic comprises at least one *Lactobacillus* species. In an embodiment, the prebiotic comprises at least one oligosaccharide. In an embodiment, the subject is a human. In an embodiment, the Extended-Spectrum Beta-Lactamases (ESBL) producing organism comprises Extended-Spectrum Beta-Lactamases (ESBL) producing gram negative bacteria. In an embodiment, the probiotic comprises *Lactobacillus plantarum.* In an embodiment, the probiotic comprises *Lactobacillus plantarum* strain ATCC202195. In an embodiment, the at least one oligosaccharide is selected from a group comprising (a) a fructo-oligosaccharide (FOS), (b) a pectin or a pectic polysaccharide, (c) a mannan, (d) a pentosan, a beta-glucan, an arabinan or a galactan, and (e) mixtures thereof. In an embodiment, the at least one oligosaccharide comprises the fructo- oligosaccharide. In an embodiment, the composition is administered by any of an oral route of administration and a parenteral route of administration. In an embodiment, the composition comprises 1-10 billion counts of cells of *Lactobacillus plantarum* strain ATCC202195 and 100-500 mg of the fructo-oligosaccharide.. In an embodiment, the composition comprises 1- 5 billion counts of cells of *Lactobacillus plantarum* strain ATCC202195 and 150-350 mg of the fructo-oligosaccharide. In an embodiment, the composition further comprises at least one pharmaceutically acceptable excipient.

Another aspect of the present disclosure relates to a method of preventing acquisition of or alleviating symptoms associated with an infection caused by an Extended- Spectrum Beta-Lactamases (ESBL) producing organism in a subject in need thereof, the method comprising administering to the subject a therapeutically effective amount of a probiotic and an effective amount of a prebiotic. In an embodiment, the administering comprises oral or parenteral administration of said probiotic and said prebiotic. In an embodiment, the method comprises administering to the subject a composition comprising the therapeutically effective amount of the probiotic and the effective amount of the prebiotic. In an embodiment, the composition further comprises at least one pharmaceutically acceptable excipient. In an embodiment, the administering comprises simultaneous, sequential or intermittent administration of the therapeutically effective amount of the probiotic and the effective amount of the prebiotic. In an embodiment, the probiotic comprises at least one *Lactobacillus* species. In an embodiment, the prebiotic comprises at least one oligosaccharide. In an embodiment, the at least one oligosaccharide is selected from a group comprising (a) a fructo-oligosaccharide (FOS), (b) a pectin or a pectic polysaccharide, (c) a mannan, (d) a pentosan, a beta-glucan, an arabinan or a galactan, and (e) mixtures thereof. In an embodiment, the therapeutically effective amount of the probiotic comprises 1-10 billion counts of cells of *Lactobacillus plantarum* strain ATCC202195. In an embodiment, the effective amount of the prebiotic comprises 100-500 mg of the fructo-oligosaccharide. In an embodiment, the therapeutically effective amount of the probiotic comprises 1-5 billion counts of cells of *Lactobacillus plantarum* strain ATCC202195. In an embodiment, the effective amount of the prebiotic comprises 150-350 mg of the fructo-oligosaccharide.

In an embodiment, said prebiotic is selected from a group consisting of (a) an oligosaccharide, (b) a fructo-oligosaccharide ("FOS"), such as a soy fructo-oligosaccharide, inulin or banana fiber, (c) a pectin or pectic polysaccharide, (d) a mannan, such as guar gum, locust bean gum, konjac, or xanthan gum, (e) a pentosan, beta-glucan, arabinan and galactan, such as larch arabinogalactan, and (f) mixtures thereof.

The compositions of the present disclosure provide a myriad of health benefits including, but not limited to, promoting gut maturation, promoting maturation of the gut nervous system, enhancing gut health, enhancing protection later in life, promoting the maturation of the immune system, contributing to support of natural defenses, contributing to support growth, and enhancing gut comfort while reducing crying time, cramps and/or colics, fulfilling at least partially the nutritional requirements of neonates and infants.

In some embodiments, the pharmaceutical composition of the present invention can be administered to children of age between 1 to 5 years and adults for prevention and treatment of infections by Extended-spectrum beta-lactamases (ESBL) producing bacteria and other drug resistant pathogens.

As utilized herein, the term "probiotic" refers to microorganisms that form at least a part of the transient or endogenous flora monoculture, and/or a mixed culture of living or dead microorganisms, spores, fractions thereof, or metabolic products thereof that exhibit a beneficial prophylactic and/or therapeutic effect on the host organism. Probiotics are beneficial bacteria that can be found in various foods, or in the form of dietary supplements.

Prebiotics are non-digestible food ingredients that can stimulate growth of intestinal bacterial growth. The pharmaceutical compositions and methods of the present invention include one or more prebiotics in combination with one or more probiotics. In certain embodiments, these one or more prebiotics include, for example and without limitation, carbohydrates or oligosaccharides and polysachharides, more preferably oligo-fructose. Sources of oligosaccharides can include fruits, legumes, and whole grains.

Fructo-oligosaccharides (FOS) are long-chain polysaccharides comprised primarily of fructose monosaccharides bonded together by 1-β-D-fructofuranosyl linkages. Upon ingestion, fructo-oligosaccharides are only partially hydrolyzed as they pass through the mouth, stomach, and small intestine. In the large intestine, they became food for certain probiotics, and are metabolized into short chain fatty acids, mainly acetic, propionic, butyric, and lactic acids. As a consequence of this fermentation, a considerable amount of bacterial mass is produced. This results in increased numbers of probiotic, a lowered intestinal pH, and is believed to inhibit pathogens. A pH decrease will increase solubility of calcium and other minerals and may enhance the absorption of calcium and magnesium. Illustrative fructo-oligosaccharides include inulin, banana fiber, and soy fructo-oligosaccharides, and are found in honey, beer, onion, asparagus, Chinese chive, maple sugar, oats, and Jerusalem artichoke.

Examples of probiotic micro-organisms can include but not limited to *Lactobacillus plantarum, Lactobacillus acidophilus, Lactobacillus alimentarius, Lactobacillus casei subsp. casei, Lactobacillus casei Shirota, Lactobacillus curvatus, Lactobacillus delbruckii subsp. lactis, Lactobacil-2Q lus farciminus, Lactobacillus gasseri,* and *Lactobacillus helveticus.* In one of the preferred embodiments of the present invention, the probiotic is *Lactobacillus plantarum.* The present probiotic includes a *Lactobacillus plantarum* strain ATCC 202195.

The growth of various *Lactobacillus* species to form cell cultures, cell pastes, and spore preparations is generally well-known within the art. The culture and preparative methods for *Lactobacillus plantarum* may be readily utilized and/or modified for growth and preparation of the other (lactic) acid-producing bacteria.

*Lactobacillus plantarum* may be utilized as a model for various other acid-producing (e.g., lactic acid) species of probiotic bacteria.

The term "pharmaceutical composition", as used herein can be construed as but not limited to a nutritional composition, a nutraceutical composition, a nutritional supplement or a pharmaceutical drug.

The term "therapeutically effective amount" as used herein means that amount of active ingredient (i.e. either probiotic or prebiotic or combination thereof) that elicits the biological or medicinal response in a subject which includes at least partial prevention or treatment of the symptoms of the disease being treated or prevented.

The term "effective amount of prebiotic" is art-recognized and used herein to denote that amount of prebiotic that supports the growth and/or maintenance of the probiotic being administered to the subject, such that the same in unison with the probiotic elicits the desired biological or medicinal response.

As used herein, the term "WP" refers to a wettable powder, which can be a powder formulation to be applied as a suspension after dispersion in water.

As used herein, the term "SE" refers to Suspo emulsion, which is a fluid heterogeneous formulation consisting of active ingredients in the form of solid particles and fine globules in continuous water phase.

As used herein, "WG" or "WDG" refer to water dispersible granules which can be defined herein as a formulation consisting of granules to be applied after disintegration and dispersion in a suitable carrier liquid.

As used herein, "SC" refers to suspension concentrates which is defined herein as a stable suspension in a fluid usually intended for dilution with water or other carrier liquid before use.

As used herein, "WP" refers to a wettable powder, which can be a powder formulation to be applied as a suspension after dispersion in water or other carrier liquid.

As used herein, "EC" refers to an emulsifiable concentrate, which can be a liquid homogenous formulation to be applied as an emulsion after dilution in water or other carrier liquid.

As used herein, "SE" refers to suspo-emulsion which is a formulation containing both solid and liquid active ingredients dispersed in an aqueous phase or other carrier liquid.

As used herein, "ZC" refers to a stable mixed formulation of capsule suspension and suspension concentrates, in fluid, normally intended for dilution with water or other carrier liquid before use.

By "promoting gut maturation" is meant in particular (but not exclusively) maturation of the digestive system, including the related nervous system and immune system.

By "enhancing gut health" or by promoting "gut comfort" is meant in particular (but not exclusively) benefits selected from contributing to better balance the intestinal flora, reduce gut permeability, reducing cramps, reducing colics, increasing gut absorption or selectivity of absorption.

By "enhancing protection later in life" is meant in particular (but not exclusively) reducing the risk of infections and/or allergies later in life. The long term effect of probiotics (for example for protection against infections or protection against atopic diseases).

By "promoting the maturation of the immune system" is meant in particular (but not exclusively) growth and development of immune system.

By "contributing to support of natural defenses" is meant in particular (but not exclusively) enhancing the immune system, fighting infection, enhancing the maturation of the immune system.

By "contributing to support growth" is meant in particular (but not exclusively) enabling the growth of the infant or children to be as close as possible to the ideal growth curve.

The phrases "parenteral administration" and "administered parenterally" as used herein refer to modes of administration other than enteral and topical administration, such as injections, and include intravenous, intramuscular, intrapleural, intravascular, intrapericardial, intraarterial, intrathecal, intracapsular, intraorbital, intracardiac, intradermal, intraperitoneal, transtracheal, subcutaneous, subcuticular, intra-articular, subcapsular, subarachnoid, intraspinal and intrastemal injection and infusion, but not limited thereto.

The phrase "pharmaceutically acceptable" is art-recognized. In certain embodiments, the term includes compositions, polymers and other materials and/or dosage forms which are, within the scope of sound medical judgment, suitable for use in contact with the tissues of mammals, human beings and animals without excessive toxicity, irritation, allergic response, or other problem or complication, commensurate with a reasonable benefit/risk ratio.

The term "treating" or "treatment" is art recognized and includes preventing a disease, disorder or condition from occurring in a human being or an animal, which may be predisposed to the disease, disorder and/or condition but has not yet been diagnosed as having it; inhibiting the disease, disorder or condition, e.g., impeding its progress; and relieving the disease, disorder, or condition, e.g., causing regression of the disease, disorder and/or condition. Treating the disease or condition includes ameliorating at least one symptom of the particular disease or condition, even if the underlying pathophysiology is not affected, is well understood in the art, and includes administration of a composition which reduces the frequency of, or delays the onset of, symptoms of a medical condition in a subject relative to a subject which does not receive such composition. The term "prophylactic or therapeutic" treatment is art-recognized and includes administration to the host of one or more of the compositions. If it is administered prior to clinical manifestation of the unwanted condition (e.g., disease or other unwanted state of the host animal) then the treatment is prophylactic, i.e., it protects the host against developing the unwanted condition, whereas if it is administered after manifestation of the unwanted condition, the treatment is therapeutic, (i.e. it is intended to diminish, ameliorate, or stabilize the existing unwanted condition or side effects thereof).

The phrase "pharmaceutically acceptable excipient" is art-recognized, and includes, for example, pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other nontoxic compatible substances employed in pharmaceutical formulations.

The present disclosure relates to a pharmaceutical composition for use in alleviating symptoms associated with infections by Extended-spectrum beta-lactamases (ESBL)-producing bacteria and other drug resistant pathogens in neonates and infants (children within the age group of 1 month to 59 months). Preferably, the present disclosure relates to a pharmaceutical composition comprising a combination of a probiotic and a prebiotic to treat infections by Extended-spectrum beta-lactamases (ESBL)-producing bacteria and other drug resistant pathogens in neonates and infants. Altogether, these health benefits include promoting gut maturation, promoting the maturation of the gut nervous system, enhancing gut health, enhancing protection later in life, promoting the maturation of the immune system, contributing to support of natural defenses, contributing to support growth, enhancing gut comfort, reducing crying time, cramps and/or colics, fulfilling at least partially the nutritional requirements of said infant..

According to a preferred embodiment of the present disclosure, said prebiotic comprises one or more of the following (a) an oligosaccharide, (b) a fructo-oligosaccharide ("FOS"), such as a soy fructo-oligosaccharide, inulin or banana fiber, (c) a pectin or pectic polysaccharide, (d) a mannan, such as guar gum, locust bean gum, konjac, or xanthan gum, (e) a pentosan, beta-glucan, arabinan and galactan, such as larch arabinogalactan, and (f) mixtures thereof.

According to an embodiment, the pharmaceutical composition of the present disclosure may be administered in solid, semi-solid, or liquid oral dosage form. In an embodiment of the present disclosure, the pharmaceutical formulation can be in the form of emulsions, solutions, suspensions, syrups, elixirs tablets, capsules, pills, granules, and suppository. In another embodiment of the present disclosure, the pharmaceutical formulation can be in the form of water dispersible granules (WG), suspension concentrates (SC), wettable powders (WP), emulsifiable concentrates (EC), granules, gel, suspo emulsions (SE), mixed formulation of capsule suspension and suspension concentrates (ZC) and the like and preferably, water dispersible granules (WG), suspo emulsions (SE) and mixed formulation of capsule suspension and suspension concentrates (ZC).

In an embodiment, the pharmaceutical formulation of the present disclosure can be dried. Drying can include spray drying, fluid bed drying, or freeze-drying. In a preferred embodiment, the pharmaceutical formulation of the present disclosure is in an orally administered dosage form of powder or granule for sachet, liquid, solution, suspension, emulsion or syrup. In another embodiment, the pharmaceutical formulation can include at least one pharmaceutically acceptable excipient selected from the group consisting of fillers, binders, diluents, thickening agents, solvents, coating agents, dispersing agents, preservatives, sweeteners, flavoring agents, antifoaming agent and stabilizers. However, those skilled in the art will appreciate that the additional pharmaceutically acceptable excipients can be used

Pharmaceutically acceptable filler may be selected from the group comprising lactose, microcrystalline cellulose, starch, pre-gelatinized starch, calcium phosphate, calcium sulfate, calcium carbonate, mannitol, sorbitol, xylitol, sucrose, maltose, fructose, dextrose, maltodextrin, and the like. Pharmaceutically acceptable binder may be selected from the group comprising starches, natural sugars, corn sweeteners, natural and synthetic gums, cellulose derivatives, gelatin, povidone, polyethylene glycol, waxes, sodium alginate, alcohols, water, and the like. Pharmaceutically acceptable diluents may be selected from the group comprising calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol, and the like. Pharmaceutically acceptable sweetener may be selected from the group comprising alitame, acesulfame potassium, aspartame, D-tryptophan, dextrose, erythritol, fructose, galactose, glycerol, glycyrrhizin, glucose, isomalt, xylitol, xylose, lactitol, lactose, levulose, maltitol, maltodextrin, maltol, maltose, mannitol, corn syrup, neohesperidin dihydrochalcone, neotame, saccharin, siclamate, sorbitol, sucralose, sucrose, tagatose, taumatin, trehalose, and the like. Pharmaceutically acceptable flavoring agent may be selected from the group comprising natural flavoring oils, anethole, acetic acid, ascorbic acid, phosphoric acid, fumaric acid, lactic acid, lemon, linalool, malic acid, menthol, eucalyptol, orange, citric acid, cinnamone, tartaric acid, thymol, vanilla, strawberry, and the like. Pharmaceutically acceptable preservative may be selected from the group comprising parabens, phenol, chlorocresol, parahydroxy benzoic acid alkyl esters, benzoic acid and salts thereof, boric acid and salts thereof, citric acid and salts thereof, sorbic acid and salts thereof, neutral preservatives, mercurial preservatives, quaternary compounds, and the like.

In yet another embodiment, the pharmaceutical composition of the present disclosure can also be administered by parenteral route such as intravenous administration. In an embodiment, the pharmaceutical composition of the present invention can include 1-10 billion count of cells of a probiotic and 100-500 mg of a prebiotic. In an embodiment, the composition comprises 1-5 billion counts of cells of *Lactobacillus plantarum* strain ATCC202195 and 150-350 mg of the fructo-oligosaccharide.

The term "an infection caused by an Extended-spectrum beta-lactamases (ESBL)-producing organism and other drug resistant pathogens" is art recognized and can include urinary tract infections (UTIs), intra-abdominal infections, primary bacteraemia, respiratory tract infections, skin & soft tissue infections, but not limited thereto. Broadly, it denotes the infection, ailment or disorder caused by one or more drug resistant pathogens such as Extended-spectrum beta-lactamases (ESBL)-producing organisms. Particularly, the compositions of the present disclosure can find its utility in preventing acquisition of and/or treatment of infections caused by drug resistant pathogens such as Extended-spectrum beta-lactamases (ESBL)-producing organisms, and particularly, by Extended-spectrum beta-lactamases (ESBL)-producing bacteria such as Extended-spectrum beta-lactamases (ESBL)-producing gram negative bacteria.

According to another embodiment, the pharmaceutical composition of the present disclosure may be administered in solid or liquid oral dosage form such as emulsions, solutions, suspensions, syrups, elixirs, tablets, chewable tablets, capsules, pills, granules, and suppository.

In an embodiment, the composition may be in the form of granules, powdered supplements (such as a supplement that can be mixed with a drink), reconstitutable powders (spray dried, dry mixed, agglomerated), ready-to-feed liquids, bars, and dilutable liquid concentrates and the likes. However, any other solid, liquid or semi-solid composition or formulation, as known to or appreciated by a person skilled in the art, can be utilized to serve its intended purpose, as laid in the present disclosure,

For parenteral administration, solutions (compositions) may be prepared using (for example) sesame or peanut oil, aqueous propylene glycol, or sterile aqueous solutions. Such solutions may be suitably buffered if necessary, and the liquid diluent is first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

The compositions of the present disclosure are preferably presented for administration to humans and animals in unit dosage forms, such as tablets, capsules, pills, powders, granules, suppositories, sterile parenteral solutions or suspensions, sterile non-parenteral solutions, suspensions, and oral solutions or suspensions and the like, containing suitable quantities of an active ingredient. For oral administration either solid or fluid unit dosage forms can be prepared.

In an embodiment, the tablet core contains one or more hydrophilic polymers. Suitable hydrophilic polymers include, but are not limited to, water swellable cellulose derivatives, polyalkylene glycols, thermoplastic polyalkylene oxides, acrylic polymers, hydrocolloids, clays, gelling starches, swelling cross-linked polymers, and mixtures thereof. Examples of suitable water swellable cellulose derivatives include, but are not limited to, sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose, hydroxyphenylcellulose, hydroxyethylcellulose (HEC), hydroxypentylcellulose, hydroxypropylethylcellulose, hydroxypropylbutylcellulose, and hydroxypropylethylcellulose, and mixtures thereof. Examples of suitable polyalkylene glycols include, but are not limited to, polyethylene glycol. Examples of suitable thermoplastic polyalkylene oxides include, but are not limited to, polyethylene oxide). Examples of suitable acrylic polymers include, but are not limited to, potassium methacrylatedivinylbenzene copolymer, polymethylmethacrylate, high-molecular weight crosslinked acrylic acid homopolymers and copolymers such as those commercially available from Noveon Chemicals under the tradename CARBOPOL^{™}. Examples of suitable hydrocolloids include, but are not limited to, alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan, and mixtures thereof. Examples of suitable clays include, but are not limited to, smectites such as bentonite, kaolin, and laponite; magnesium trisilicate; magnesium aluminum silicate; and mixtures thereof. Examples of suitable gelling starches include, but are not limited to, acid hydrolyzed starches, swelling starches such as sodium starch glycolate and derivatives thereof, and mixtures thereof. Examples of suitable swelling cross-linked polymers include, but are not limited to, cross-linked polyvinyl pyrrolidone, cross-linked agar, and cross-linked carboxymethylcellulose sodium, and mixtures thereof.

The carrier may contain one or more suitable excipients for the formulation of tablets. Examples of suitable excipients include, but are not limited to, fillers, adsorbents, binders, disintegrants, lubricants, glidants, release-modifying excipients, superdisintegrants, antioxidants or mixtures thereof.

Suitable binders include, but are not limited to, dry binders such as polyvinyl pyrrolidone and hydroxypropylmethylcellulose; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, polyvinyl pyrrolidone, cellulosics, sucrose, and starches; and mixtures thereof. Suitable disintegrants include, but are not limited to, sodium starch glycolate, cross-linked polyvinylpyrrolidone, cross-linked carboxymethylcellulose, starches, microcrystalline cellulose, and mixtures thereof. Suitable lubricants include, but are not limited to, long chain fatty acids and their hydrates or solvates, such as magnesium stearate and stearic acid, talc, glycerides waxes, and mixtures thereof. Suitable glidants include, but are not limited to, colloidal silicon dioxide. Suitable release-modifying excipients include, but are not limited to, insoluble edible materials, pH-dependent polymers, and mixtures thereof.

Suitable insoluble edible materials for use as release-modifying excipients include, but are not limited to, water-insoluble polymers and low-melting hydrophobic materials, copolymers thereof, and mixtures thereof. Examples of suitable water-insoluble polymers include, but are not limited to, ethylcellulose, polyvinyl alcohols, polyvinyl acetate, polycaprolactones, cellulose acetate and its derivatives, acrylates, methacrylates, acrylic acid copolymers, copolymers thereof and mixtures thereof. Suitable low-melting hydrophobic materials include, but are not limited to, fats, fatty acid esters, phospholipids, waxes, and mixtures thereof. Examples of suitable fats include, but are not limited to, hydrogenated vegetable oils such as for example cocoa butter, hydrogenated palm kernel oil, hydrogenated cottonseed oil, hydrogenated sunflower oil, and hydrogenated soybean oil, free fatty acids and their hydrates or solvates, and mixtures thereof. Examples of suitable fatty acid esters include, but are not limited to, sucrose fatty acid esters, mono-, di-, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, stearoyl macrogol-32 glycerides, and mixtures thereof. Examples of suitable phospholipids include phosphotidyl choline, phosphotidyl serene, phosphotidyl enositol, phosphotidic acid, and mixtures thereof. Examples of suitable waxes include, but are not limited to, carnauba wax, spermaceti wax, beeswax, candelilla wax, shellac wax, microcrystalline wax, and paraffin wax; fat-containing mixtures such as chocolate, and mixtures thereof. Examples of super disintegrants include, but are not limited to, croscarmellose sodium, sodium starch glycolate and cross-linked povidone (crospovidone). In one embodiment the tablet core contains up to about 5 percent by weight of such super disintegrant.

Examples of antioxidants include, but are not limited to, tocopherols, ascorbic acid, sodium pyrosulfite, butylhydroxytoluene, butylated hydroxyanisole, edetic acid, and edetate hydrates or solvates, and mixtures thereof. Examples of preservatives include, but are not limited to, citric acid, tartaric acid, lactic acid, malic acid, acetic acid, benzoic acid, and sorbic acid, and mixtures thereof.

In one embodiment, the immediate release coating has an average thickness of at least 50 microns, such as from about 50 microns to about 2500 microns; e.g., from about 250 microns to about 1000 microns. In embodiment, the immediate release coating is typically compressed at a density of more than about 0.9 g/cc, as measured by the weight and volume of that specific layer.

In one embodiment, the immediate release coating contains a first portion and a second portion, wherein at least one of the portions contains the second pharmaceutically active agent. In one embodiment, the portions contact each other at a center axis of the tablet. In one embodiment, the first portion includes the first pharmaceutically active agent and the second portion includes the second pharmaceutically active agent.

In one embodiment, the first portion contains the first pharmaceutically active agent and the second portion contains the second pharmaceutically active agent. In one embodiment, one of the portions contains a third pharmaceutically active agent. In one embodiment one of the portions contains a second immediate release portion of the same pharmaceutically active agent as that contained in the tablet core.

In one embodiment, the outer coating portion is prepared as a dry blend of materials prior to addition to the coated tablet core. In another embodiment the outer coating portion is included of a dried granulation including the pharmaceutically active agent.

Formulations with different drug release mechanisms described above could be combined in a final dosage form containing single or multiple units. Examples of multiple units include multilayer tablets, capsules containing tablets, beads, or granules in a solid or liquid form. Typical, immediate release formulations include compressed tablets, gels, films, coatings, liquids and particles that can be encapsulated, for example, in a gelatin capsule. Many methods for preparing coatings, covering or incorporating drugs, are known in the art.

The immediate release dosage, unit of the dosage form, i.e., a tablet, a plurality of drug-containing beads, granules or particles, or an outer layer of a coated core dosage form, contains a therapeutically effective quantity of the active agent with conventional pharmaceutical excipients. The immediate release dosage unit may or may not be coated, and may or may not be admixed with the delayed release dosage unit or units (as in an encapsulated mixture of immediate release drug-containing granules, particles or beads and delayed release drug-containing granules or beads).

Extended release formulations are generally prepared as diffusion or osmotic systems, for example, as described in "Remington-The Science and Practice of Pharmacy", 20th. Ed., Lippincott Williams & Wilkins, Baltimore, Md., 2000. A diffusion system typically consists of one of two types of devices, reservoir and matrix, which are well known and described in the art. The matrix devices are generally prepared by compressing the drug with a slowly dissolving polymer carrier into a tablet form.

An immediate release portion can be added to the extended release system by means of either applying an immediate release layer on top of the extended release core; using coating or compression processes or in a multiple unit system such as a capsule containing extended and immediate release beads.

Delayed release dosage formulations are created by coating a solid dosage form with a film of a polymer, which is insoluble in the acid environment of the stomach, but soluble in the neutral or slightly basic environment of small intestine. The delayed release dosage units can be prepared, for example, by coating a drug or a drug-containing composition with a selected coating material. The drug-containing composition may be a tablet for incorporation into a capsule, a tablet for use as an inner core in a "coated core" dosage form, or a plurality of drug-containing beads, particles or granules, for incorporation into either a tablet or capsule.

A pulsed release dosage form is one that mimics a multiple dosing profile without repeated dosing and typically allows at least a twofold reduction in dosing frequency as compared to the drug presented as a conventional dosage form (e.g., as a solution or prompt drug-releasing, conventional solid dosage form). A pulsed release profile is characterized by a time period of no release (lag time) or reduced release followed by rapid drug release.

Each dosage form contains any or a combination of a therapeutically effective amount of probiotic and an effective amount of prebiotic (each of the "probiotic" and "prebiotic", independently, are alternatively and synonymously referred to as "active agent" or "active ingredient" herein through the present disclosure). In one embodiment of dosage forms that mimic a twice daily dosing profile, approximately 30 wt. % to 70 wt. %, preferably 40 wt. % to 60 wt. %, of the total amount of active agent in the dosage form is released in the initial pulse, and, correspondingly approximately 70 wt. % to 30 wt. %, preferably 60 wt. % to 40 wt. %, of the total amount of active agent in the dosage form is released in the second pulse. For dosage forms mimicking the twice daily dosing profile, the second pulse is preferably released approximately 3 hours to less than 14 hours, and more preferably approximately 5 hours to 12 hours, following administration.

Another dosage form contains a compressed tablet or a capsule having a drug-containing immediate release dosage unit, a delayed release dosage unit and an optional second delayed release dosage unit. In this dosage form, the immediate release dosage unit contains a plurality of beads, granules particles that release drug substantially immediately following oral administration to provide an initial dose. The delayed release dosage unit contains a plurality of coated beads or granules, which release drug approximately 3 hours to 14 hours following oral administration to provide a second dose.

Methods of preparing various pharmaceutical compositions of the present disclosure are well within the reach of those skilled in this art. For examples of methods of preparing pharmaceutical compositions, see Remington's Pharmaceutical Sciences, Mack Publishing Company, Easton, Pa., 19th Edition (1995).

In addition, in certain embodiments, subject compositions of the present application maybe lyophilized or subjected to another appropriate drying technique such as spray drying. The subject compositions may be administered once, or may be divided into a number of smaller doses to be administered at varying intervals of time, depending in part on the release rate of the compositions and the desired dosage.

Methods of preparing these formulations or compositions include the step of bringing into association compound with the carrier and, optionally, one or more accessory ingredients. In general, the formulations are prepared by uniformly and intimately bringing into association a compound with liquid carriers, or finely divided solid carriers, or both, and then, if necessary, shaping the product.

In solid dosage forms for oral administration (capsules, tablets, pills, dragees, powders, granules and the like), the subject composition is mixed with one or more pharmaceutically acceptable carriers and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using lactose or milk sugars, as well as high molecular weight polyethylene glycols and the like.

Liquid dosage forms for oral administration include pharmaceutically acceptable emulsions, microemulsions, solutions, suspensions, syrups and elixirs. In addition to the subject compositions, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, corn, peanut, sunflower, soybean, olive, castor, and sesame oils), glycerol, tetrahydrofuryl alcohol, polyethylene glycols and fatty acid esters of sorbitan, and mixtures thereof.

Suspensions, in addition to the active ingredient, may contain suspending agents such as, for example, ethoxylated isostearyl alcohols, polyoxyethylene sorbitol, and sorbitan esters, microcrystalline cellulose, aluminum metahydroxide, bentonite, agar-agar and tragacanth, or mixtures thereof.

In an embodiment, each of the prebiotic and probiotic can be prepared as separate formulations (such as tablets, capsules, powder and the likes) and the same can be orally administered to the subject in need thereof. Each of the prebiotic and probiotic can be administered (such as orally ingested) simultaneously, sequentially or intermittently so far the beneficial effects of each of the prebiotic and the probiotic are maintained, as disclosed herein. It should also be appreciated that any other active ingredient, herb, neutraceutical, or excipient, as known to or appreciated by a person skilled in the pertinent art, can be co-administered with the composition(s) and/or included in the compositions of the present disclosure, The term "co-administer" means to administer more than one active agent, such that the duration of physiological effect of one active overlaps with the physiological effect of a second agent. In some embodiments, co-administration includes administering one agent within 0.5, 1, 2, 4, 6, 8, 10, 12, 16, 20, or 24 hours of a second agent. Co-administration includes administering two agents simultaneously, approximately simultaneously (e.g., within about 1, 5, 10, 15, 20, or 30 minutes of each other), or sequentially in any order. In some embodiments, co-administration can be accomplished by co-formulation, i.e., preparing a single pharmaceutical composition including both agents. In other embodiments, the agents can be formulated separately. In another embodiment, the agents may be linked or conjugated to one another. In one embodiment, the prebiotic and the probiotic are formulated into a single formulation (such as a tablet, capsule or powder that includes both the required dosage of prebiotic as well as the required dosage of probiotic). The formulation that includes both the required dosage of prebiotic as well as the required dosage of probiotic can be a single dosage formulation or a multiple dosage formulation.

In an embodiment, a combination of 1 billion count of cells of L. plantarum and about 150 mg of fructo-oligosaccharides, with or without one or a combination of excipients such as maltodextrin, is mixed with 1-2 ml of saline solution or dextrose saline solution (5% DNS) or other suitable carrier liquid and can be orally fed once a day to a newborn infant starting from day 1 (i.e. orally fed to human infant of age of 1 day) and fed daily for 7 days (one week). The same treatment regimen can be followed for 2^{nd} Month i.e. a mixture of 1 billion count of cells of L. plantarum and about 150 mg of fructo-oligosaccharides, with or without one or a combination of excipients such as maltodextrin, can be mixed with 1-2 ml of saline solution or dextrose saline solution (5% DNS) or other suitable carrier liquid and can be orally fed once a day for one week. For 3^{rd}, 4^{th} and 5^{th} Month, a mixture of 2 billion count of cells of L. plantarum and about 150 mg of fructo-oligosaccharides, with or without one or a combination of excipients such as maltodextrin, can be mixed with 1-2 ml of saline solution or dextrose saline solution (5% DNS) or other suitable carrier liquid and can be orally fed once a day for one week. For 6^{th} Month through 12^{th} Month, a mixture of 4 billion count of cells of L. plantarum and about 300 mg of fructo-oligosaccharides, with or without one or a combination of excipients such as maltodextrin, can be mixed with 3 ml of saline solution or dextrose saline solution (5% DNS) or other suitable carrier liquid and can be orally fed once a day for one week. For 13^{th} Month through 24^{th} Month, a mixture of 5 billion count of cells of L. plantarum and about 500 mg of fructo-oligosaccharides, with or without one or a combination of excipients such as maltodextrin, can be mixed with 5 ml of saline solution or dextrose saline solution (5% DNS) or other suitable carrier liquid and can be orally fed once a day for one week. Alternatively, the composition(s) of the present disclosure can be administered following any other suitable treatment regimen to derive the beneficial effects as disclosed in the present application, without departing from the scope and spirit of the present disclosure. Appropriateness of such treatment regimen can be determined and/or suitable treatment regimen can be selected (including dosage, frequency of administration, route of administration, or any change therein) by a medical practitioner based on one or a combination of parameters such as age of the child/infant, ethnicity, co-morbidity (such as diarrhea, vomiting and the likes), patient compliance and the likes and all such variations in the therapeutic/treatment regimen are to be treated as part and parcel of the present disclosure without departing from the scope and spirit of the present invention.

The composition of the present disclosure provides a myriad of health benefits including, but not limited to, promoting gut maturation, promoting the maturation of the gut nervous system, enhancing gut health, enhancing protection later in life, promoting the maturation of the immune system, contributing to support of natural defences, contributing to support growth, enhancing gut comfort, reducing crying time, cramps and/or colics, fulfilling at least partially the nutritional requirements of infant.

While the foregoing describes various embodiments of the invention, other and further embodiments of the invention may be devised without departing from the basic scope thereof. The scope of the invention is determined by the claims that follow. The invention is not limited to the described embodiments, versions or examples, which are included to enable a person having ordinary skill in the art to make and use the invention when combined with information and knowledge available to the person having ordinary skill in the art.

### EXAMPLES

### Compositions including Lactobacillus plantarum ATCC 202195 and fructo-oligosaccharides

Powdered formulations, as shown in Table 1 below, each including *Lactobacillus plantarum* ATCC 202195, fructo-oligosaccharides and maltodextrin were prepared.

**Table 1: Powdered Formulations**

| **Formulation No.** | ***L. plantarum* ATCC 202195 (count of cells in billions per formulation)** | **Fructo-oligosaccharides (mg per formulation)** | **maltodextrin (mg per formulation)** |
|---|---|---|---|
| 1 | 1 | 150 | 350 |
| 2 | 2 | 150 | 350 |
| 3 | 4 | 300 | 350 |

Each of the aforesaid formulations 1 through 3 were packed in sachets and stored in cool moisture proof containers for further usage.

### Administration of Lactobacillus plantarum ATCC 202195 and fructo-oligosaccharides to infants

A cohort of infants were orally fed with a synbiotic preparation containing *Lactobacillus plantarum* ATCC 202195 and fructo-oligosaccharides after birth. Neonates were enrolled from over 70 community sites and at two tertiary care centers, Capital Hospital, Bhubaneswar and SCB Medical College, Cuttack, in the state of Orissa, India. All infants were of more than 35 weeks gestation and more than 1800 grams weight at birth. The group designated as "community infants" were born via normal vaginal delivery and then were discharged within 24-48 hours of life. The hospital-born infants were delivered via Cesarean section and remained in the hospital for 6-7 days as per hospital protocol. After enrollment, neonates were randomized to either symbiotic-treated (orally fed with *L. plantarum* and Fructo-oligosaccharides) or placebo, and were orally fed within the first 24 hours of life, followed by oral administration daily for 7 days of life. Each of the infants (treatment group, referred to herein as "symbiotic-treated") was orally fed with formulation 1 mixed with 1-2 ml of 5% dextrose saline solution (5% DNS) once a day starting from day 1 (i.e. orally fed to human infant of age of 1 day) for 7 days (one week). The same treatment was repeated for 2^{nd} Month i.e. orally fed with formulation 1 mixed with 1-2 ml of 5% dextrose saline solution (5% DNS) once a day for one week. For 3^{rd}, 4^{th} and 5^{th} Month, each of the infants were orally fed with formulation 2 mixed with 1-2 ml of 5% dextrose saline solution (5% DNS) once a day for one week in each of 3^{rd}, 4^{th} and 5^{th} month. For 6^{th} Month through 12^{th} Month, each of the infants were orally fed with formulation 3 mixed with 3 ml of 5% dextrose saline solution (5% DNS) once a day for one week in each of 6^{th} through 12^{th} month. Stool samples were randomly selected (for each of the symbiotic-treated and placebo groups) at three time-points: 2/3 months, 5/6 months and 9/12 months for examination.

### Analysis of Bacterial Isolates

Stool samples were cultured on primary stool culture media for isolation of bacterial organisms. No selective media was used. Gram-negative isolates were identified by rapid tests (BioMeiurix). Table 1 below provides primer sequences used for multiplex PCR.

**Table 1: Primer sequences used for multiplex PCR**

| ESBL gene-type | Primer-Sequence (5'-3') | Tₘ in °C | PCR-Amplicons | Reference |
|---|---|---|---|---|
| SHV | fwd: tggttatgcgttatattcgcc | 62 | 867 bp | Jiang et al. 2006 |
| | rev: gcttagcgttgccagtgct | 56 | | |
| TEM | fwd: tcgccgcatacactattctcagaatga | 53 | 445 bp | Monstein et al. 2007 |
| | rev: acgctcaccggctccagatttat | 52 | | |
| CTX-M | fwd: atgtgcagyaccagtaargtkatggc | 54 | 593 bp | Monstein et al. 2007 |
| | rev: tgggtraartargtsaccagaaycagcgg | 58 | | |

### Molecular Characterization of ESBL phenotypes

*DNA isolation:* Direct DNA was also purified from infant stool using commercial kits (Qiagen Inc.).

*ESBL gene multiplex-PCR:* For the simultaneous detection of the ESBL genes, published primer pairs targeting the three ESBL genes (*SHV, CTX-M and TEM*) were used in the PCR assay (Jiang et al., Detection of extended-spectrum β-lactamases in clinical isolates of Pseudomonas aeruginosa. Antimicrob Ag Chemother. 2006, 50:2990-2995; Monstein et al., Multiplex PCR amplification assay for the detection of blaTEM, blaSHV and blaCTX-M genes in Enterobacteriaceae. APMIS. 2007, 115: 1400-08, contents of both incorporated herein in their entirety by way of reference). These specific primer pairs amplified 867, 593 & 445 bp gene targets, respectively. Each 25 µl PCR reaction utilized 2 µl of the DNA extract. Amplification steps involved initial denaturation at 94°C for 3 min.; 35 cycles of denaturation at 94°C for 30 seconds, annealing at 58°C for 30 seconds, extension at 72°C for 1 minute, followed by a final extension at 72°C for 5 minutes. Stool samples collected were assayed for presence of ESBL and the results (number of samples with presence of ESBL) are presented in Table 2A and 2B below.

**Table 2A: Analysis of stool samples for presence of ESBL**

| **Time (Months)** | **Community-born infants** | | |
|---|---|---|---|
| | **Placebo (n=25)** | **Probiotic (n=25)** | **P (Fisher's Exact)** |
| 2/3 Months | 21 (84%) | 14 (56%) | 0.031 |
| 5/6 Months | 15 (60%) | 21 (84%) | 0.057 |
| 9/12 Months | 20 (80%) | 17 (68%) | 0.260 |

**Table 2B: Analysis of stool samples for presence of ESBL**

| **Time (Months)** | **Hospital-born infants** | | |
|---|---|---|---|
| | **Placebo (n=23)** | **Probiotic (n=46)** | **P (Fisher's Exact)** |
| 2/3 Months | 18 (78.26%) | 29 (63.04%) | 0.158 |
| 5/6 Months | 22 (95.65%) | 34 (73.91%) | 0.026 |
| 9/12 Months | 21 (91.30%) | 27 (58.69%) | 0.004 |

In conclusion, significant reduction in the acquisition of ESBL gene-harboring bacteria (specifically, ESBL producing gram-negative bacteria) in both hospital-born and community-born infants upon treatment with the synbiotic was observed as compared to the placebo recipients. These surprising results demonstrated for the first time that oral administration of synbiotics, and specifically, a combination of *Lactobacillus plantarum* (strain ATCC202195) and a prebiotic, to infants early in life reduces the acquisition of ESBL gene-harboring bacteria (specifically, ESBL producing gram-negative bacteria) in both hospital-born and community-born infants.

### ADVANTAGES

The present disclosure provides a pharmaceutical composition including a combination of a prebiotic and a probiotic.

The present disclosure provides a pharmaceutical composition effective against prevention and treatment of infections caused by Extended spectrum beta-lactamases (ESBL)-producing organisms and other drug resistant pathogens.

The present disclosure provides a pharmaceutical composition that promotes gut maturation and enhances gut health and protection later in life in neonates and infants.

The present disclosure provides a pharmaceutical composition that promotes maturation of the immune system and contributes to support of natural defences to enhance gut comfort while reducing crying time, cramps and/or colics in neonates and infants.

## Claims

1. A pharmaceutical composition for use in preventing acquisition of or for use in alleviating symptoms associated with an infection caused by an Extended-Spectrum Beta-Lactamases (ESBL) producing organism in a subject, the composition comprising:
a therapeutically effective amount of a probiotic; and
an effective amount of a prebiotic;
wherein said probiotic is *Lactobacillus plantarum* strain ATCC202195.

2. The composition for use as claimed in claim 1, wherein said prebiotic comprises at least one oligosaccharide.

3. The composition for use as claimed in claim 1, wherein said subject is a human and wherein said Extended-Spectrum Beta-Lactamases (ESBL) producing organism comprises Extended-Spectrum Beta-Lactamases (ESBL) producing gram negative bacteria.

4. The composition for use as claimed in claim 2, wherein said at least one oligosaccharide is selected from the group consisting of (a) a fructo-oligosaccharide (FOS), (b) a pectin or a pectic polysaccharide, (c) a mannan, (d) a pentosan, a beta-glucan, an arabinan or a galactan, and (e) mixtures thereof.

5. The composition for use as claimed in claim 2, wherein said at least one oligosaccharide comprises fructo-oligosaccharide.

6. The composition for use as claimed in claim 1, wherein said composition is administered by any of an oral route of administration and a parenteral route of administration.

7. The composition for use as claimed in claim 1, wherein the therapeutically effective amount of the probiotic comprises 1-10 billion counts of cells of *Lactobacillus plantarum* strain ATCC202195 and the effective amount of the prebiotic comprises 100-500 mg of fructo-oligosaccharide.

8. The composition for use as claimed in claim 1, wherein said composition further comprises at least one pharmaceutically acceptable excipient.

## Patentansprüche

1. Pharmazeutische Zusammensetzung zur Verwendung bei der Vermeidung des Erwerbs oder zur Verwendung bei der Linderung von Symptomen, die mit einer Infektion assoziiert sind, die durch einen Extended-Spectrum-Beta-Lactamasen (ESBL) produzierenden Organismus in einem Individuum verursacht wird, wobei die Zusammensetzung umfasst:
eine therapeutisch wirksame Menge eines Probiotikums; und
eine wirksame Menge eines Präbiotikums;
wobei das Probiotikum der Stamm ATCC202195 von *Lactobacillus plantarum* ist.

2. Zusammensetzung zur Verwendung nach Anspruch 1, wobei das Präbiotikum mindestens ein Oligosaccharid umfasst.

3. Zusammensetzung zur Verwendung nach Anspruch 1, wobei das Individuum ein Mensch ist und wobei der die Extended-Spectrum-Beta-Lactamasen (ESBL) produzierende Organismus Extended-Spectrum-Beta-Lactamasen (ESBL) produzierende gramnegative Bakterien umfasst.

4. Zusammensetzung zur Verwendung nach Anspruch 2, wobei das mindestens eine Oligosaccharid ausgewählt ist aus der Gruppe bestehend aus (a) einem Fructo-Oligosaccharid (FOS), (b) einem Pektin oder einem pektischen Polysaccharid, (c) einem Mannan, (d) einem Pentosan, einem Beta-Glucan, einem Arabinan oder einem Galactan und (e) Mischungen davon.

5. Zusammensetzung zur Verwendung nach Anspruch 2, wobei das mindestens eine Oligosaccharid Fructo-Oligosaccharid umfasst.

6. Zusammensetzung zur Verwendung nach Anspruch 1, wobei die Zusammensetzung auf beliebigen von einem oralen Verabreichungsweg und einem parenteralen Verabreichungsweg verabreicht wird.

7. Zusammensetzung zur Verwendung nach Anspruch 1, wobei die therapeutisch wirksame Menge des Probiotikums eine Zellanzahl von 1-10 Milliarden Zellen des Stammes ATCC202195 von *Lactobacillus plantarum* umfasst und die wirksame Menge des Präbiotikums 100-500 mg Fructo-Oligosaccharid umfasst.

8. Zusammensetzung zur Verwendung nach Anspruch 1, wobei die Zusammensetzung ferner mindestens einen pharmazeutisch akzeptablen Hilfsstoff umfasst.

## Revendications

1. Composition pharmaceutique destinée à être utilisée dans la prévention de l'acquisition ou dans le soulagement de symptômes associés à une infection provoquée par un organisme producteur de bêta-lactamases à spectre étendu (BLSE) chez un sujet, la composition comprenant :
une quantité thérapeutiquement efficace d'un probiotique ; et
une quantité efficace d'un prébiotique ;
dans laquelle ledit probiotique est la souche ATCC202195 de *Lactobacillus plantarum.*

2. Composition destinée à être utilisée selon la revendication 1, dans laquelle ledit prébiotique comprend au moins un oligosaccharide.

3. Composition destinée à être utilisée selon la revendication 1, dans laquelle ledit sujet est un être humain et dans laquelle ledit organisme producteur de bêta-lactamases à spectre étendu (BLSE) comprend des bactéries gram négatives productrices de bêta-lactamases à spectre étendu (BLSE).

4. Composition destinée à être utilisée selon la revendication 2, dans laquelle ledit au moins un oligosaccharide est choisi dans le groupe constitué (a) d'un fructo-oligosaccharide (FOS), (b) d'une pectine ou d'un polysaccharide pectique, (c) d'un mannane, (d) d'un pentosane, d'un bêta-glucane, d'un arabinane ou d'un galactane, et (e) de mélanges de ceux-ci.

5. Composition destinée à être utilisée selon la revendication 2, dans laquelle ledit au moins un oligosaccharide comprend un fructo-oligosaccharide.

6. Composition destinée à être utilisée selon la revendication 1, dans laquelle ladite composition est administrée par l'une quelconque d'une voie d'administration orale et d'une voie d'administration parentérale.

7. Composition destinée à être utilisée selon la revendication 1, dans laquelle la quantité thérapeutiquement efficace du probiotique comprend 1 à 10 milliards de cellules de la souche ATCC202195 de *Lactobacillus plantarum* et la quantité efficace du probiotique comprend 100 à 500 mg de fructo-oligosaccharide.

8. Composition destinée à être utilisée selon la revendication 1, dans laquelle ladite composition comprend en outre au moins un excipient pharmaceutiquement acceptable.
